(19) Europäisches Patentamt
European Patent Office
Office européen des brevets

(11) **EP 4 279 056 A1**

(12) **EUROPEAN PATENT APPLICATION**
published in accordance with Art. 153(4) EPC

(43) Date of publication:
**22.11.2023 Bulletin 2023/47**

(21) Application number: **22739490.5**

(22) Date of filing: **14.01.2022**

(51) International Patent Classification (IPC):
***A61K 8/02*** *(2006.01)* ***A61K 8/19*** *(2006.01)*
***A61K 8/25*** *(2006.01)* ***A61K 8/31*** *(2006.01)*
***A61Q 1/12*** *(2006.01)*

(52) Cooperative Patent Classification (CPC):
**A61K 8/02; A61K 8/19; A61K 8/25; A61K 8/29; A61K 8/31; A61K 8/86; A61K 8/92; A61Q 1/02; A61Q 1/12**

(86) International application number:
**PCT/JP2022/001135**

(87) International publication number:
**WO 2022/154085 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.01.2021 JP 2021004558**

(71) Applicant: **Kao Corporation**
**Chuo-ku**
**Tokyo 103-8210 (JP)**

(72) Inventors:
- **ABE, Tomoaki**
  **Odawara-shi, Kanagawa 250-0002 (JP)**
- **YAMAWAKI, Kenji**
  **Odawara-shi, Kanagawa 250-0002 (JP)**
- **OTA, Ko**
  **Odawara-shi, Kanagawa 250-0002 (JP)**

(74) Representative: **Hoffmann Eitle**
**Patent- und Rechtsanwälte PartmbB**
**Arabellastraße 30**
**81925 München (DE)**

# (54) METHOD FOR PRODUCING SOLID POWDERED COSMETIC

(57) The present invention provides a method for producing a solid powder cosmetic which can contain an oil agent in a large amount, simultaneously satisfies both the dispersibility and the glittering property of a pigment, and besides, is excellent in use impression. The present invention relates to a method for producing a solid powder cosmetic, the method including the following steps (A) to (C): (A) a step of mixing an oil agent, a powder, an aqueous solvent having an SP value of 21 $(cal/cm^3)^{1/2}$ or higher, and a surfactant in an amount of 0 to 10 mass% with respect to the oil agent, and mixing the mixture at a circumferential speed of a stirring blade tip of 6 to 39 m/s to thereby make a slurry; (B) a step of packing a cosmetic in a slurry form obtained in the step (A) in a cosmetic container; and (C) a step of, after the step (B), removing the solvent from the cosmetic in the slurry form.



Processed by Luminess, 75001 PARIS (FR)

## Description

### Field of the Invention

The present invention relates to a method for producing a solid powder cosmetic.

### Background of the Invention

Methods for producing solid powder cosmetics are divided into dry methods including compression molding an oil powder bulk obtained by mixing a powder and an oil agent, and wet methods including adding water or a volatile solvent such as an alcohol to an oil powder bulk to make a slurry, packing the slurry in a container, and thereafter, removing the volatile solvent through drying to thereby obtain cosmetics. The cosmetics obtained by the wet methods are characterized by having the fine powder texture and the moist use impression due to the improved homogeneous dispersion of the powder and oil agent, as compared with those obtained by the dry methods.

Also in the wet method, however, a problem is that sufficient homogeneous dispersion of a powder and an oil agent is highly difficult, and the use impression is impaired by agglomerates of the powder and the oil agent. Further, when a large amount of the oil agent is blended, the oil agent oozes out in a step of mixing the powder and the oil agent and becomes unable to be handled, and therefore the amount of the oil agent to be blended has been limited. As a method of improving the dispersibility of an oil agent and a pigment, a method including mixing a powder component and an oily component as a binder in an organic solvent by using a medium stirring mill to make a slurry has been reported (Patent Literature 1). Further, a method involving using a specific oil agent that is dissolved in ethanol to improve the dispersibility of the oil agent has been reported (Patent Literature 2).

Although these methods improve the dispersibility of the oil agent, the oil agent is dissolved in a dispersion solvent, and thus may be removed together with the solvent upon solvent suction in the molding process. Hence, a problem is that a sufficient amount of the oil agent cannot be left in products. Another problem is that the use of a mill or the like improves the dispersibility of the pigment, but results in reduced glitter due to crushing of a glittering pigment having a high aspect ratio.

Patent Literature 1: JP-A-2001-213722
Patent Literature 2: JP-A-2013-209316

### Summary of the Invention

The present invention provides a method for producing a solid powder cosmetic, the method comprising the following steps (A) to (C):

(A) a step of mixing an oil agent, a powder, an aqueous solvent having an SP value of 21 $(cal/cm^3)^{1/2}$ or higher, and a surfactant in an amount of 0 to 10 mass% with respect to the oil agent, and stirring the mixture at a circumferential speed of a stirring blade tip of 6 m/s or higher and 39 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

### Detailed Description of the Invention

The present invention provides a method for producing a solid powder cosmetic which can contain a large amount of an oil agent, simultaneously satisfies both the dispersibility and the glittering property of a pigment, and besides, is excellent in use impression.

The present inventors found that, when an oil agent and a powder are mixed in an aqueous solvent which has a specific SP value and which does not dissolve the oil agent completely, limiting the content of a surfactant to not more than a specific amount and carrying out the mixing at a stirring force higher than usual to make a slurry, and then packing the slurry in a cosmetic container and removing the solvent can provide a solid powder cosmetic which can contain the oil agent at a large amount, can have the oil agent left sufficiently in products, is good in the dispersibility of the pigment, has no agglomerate, and is excellent in the glittering property and excellent in use impression.

According to the present invention, a solid powder cosmetic can be produced which can contain a large amount of an oil agent, can have the oil agent left sufficiently in products, is good in the dispersibility of the pigment, has no agglomerate, and is excellent in the glittering property and excellent in use impression.

The method for producing a solid powder cosmetic of the present invention is characterized by including the above step (A), step (B) and step (c). In the below, each step will be described.

The step (A) is a step of mixing an oil agent, a powder, and an aqueous solvent having an SP value of 21 $(cal/cm^3)^{1/2}$ or higher, and stirring the mixture at a circumferential speed of a stirring blade tip of 6 m/s or higher and 39 m/s or lower to thereby make a slurry.

Here, in the step (A), the oil agent, the powder, the aqueous solvent and a surfactant in an amount of 0 to 10 mass% with respect to the oil agent are mixed and stirred to make a slurry. At this time, it is preferable that the oil agent and the powder are not mixed previously, in view of good dispersibility of the pigment, no agglomerate and a further improvement in the glittering property.

Examples of the oil agent to be used include hydrocarbon oils, ester oils, ether oils, silicone oils and higher alcohols having 10 to 24 carbon atoms.

More specifically, the hydrocarbon oils include squalane and mineral oils (liquid paraffin, liquid isoparaffin, heavy liquid isoparaffin). Among these hydrocarbon oils, it is preferable to contain at least one or more selected from the group consisting of liquid paraffin, liquid isoparaffin and squalane, and it is more preferable to contain at least one selected from the group consisting of liquid paraffin and squalane, in view of providing the solid powder cosmetic which is good in the dispersibility of the pigment, has no agglomerate, and is excellent in the glittering property and excellent in use impression.

The ester oils include monoester oils, diester oils, trieste oils and tetraester oils.

The monoester oils include monoesters of aliphatic or aromatic monocarboxylic acids or dicarboxylic acids having 2 to 24 carbon atoms; and specific examples thereof include cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate, octyl palmitate, 2-hexyldecyl palmitate, butyl stearate, isocetyl stearate, isocetyl isostearate, decyl oleate, isodecyl benzoate, octyl methoxycinnamate, hexyldecyl dimethyloctanoate, cetyl lactate, myristyl lactate, lanolin acetate, 2-ethylhexyl succinate, 2-hexyldecyl adipate and alkyl (C12-C15) benzoates. Among these, it is preferable to contain at least one or more selected from the group consisting of cetyl ethylhexanoate, isononyl isononanoate, isotridecyl isononanoate, isopropyl myristate and octyl methoxycinnamate; it is more preferable to contain at least one or more selected from the group consisting of cetyl ethylhexanoate, isononyl isononanoate and isotridecyl isononanoate; and it is further more preferable to contain at least one or more selected from the group consisting of cetyl ethylhexanoate and isononyl isononanoate, in view of providing the solid powder cosmetic which is good in the dispersibility of the pigment, has no agglomerate, and is excellent in the glittering property and excellent in use impression.

The diester oils include diesters of dicarboxylic acids having 3 to 18 carbon atoms, and di-fatty acid esters of polyhydric alcohols. Specific examples thereof include propylene glycol dicaprylate, neopentyl glycol dicaprate, glycol distearate, propylene glycol diisostearate, glyceryl diisostearate, glyceryl monoisostearate monomyristate, glycerol di-2-heptylundecanoate, di-2-ethylhexyl succinate, diisopropyl sebacate, diisostearyl malate, ethylene glycol di-2-ethylhexanoate, diisobutyl adipate, di-2-heptylundecyl adipate and di-2-ethylhexyl sebacate. Among these, it is preferable to contain at least one or more selected from the group consisting of propylene glycol dicaprylate, neopentyl glycol dicaprate, glycol distearate, diisostearyl malate and propylene glycol diisostearate; it is more preferable to contain at least one or more selected from the group consisting of propylene glycol dicaprylate, neopentyl glycol dicaprate and diisostearyl malate; it is further more preferable to contain at least one or more neopentyl glycol dicaprate and diisostearyl malate; and it is even more preferable to contain diisostearyl malate, in view of providing the solid powder cosmetic which is good in the dispersibility of the pigment, has no agglomerate, and is excellent in the glittering property and excellent in use impression.

The triester oils include tri-fatty acid esters of tri- or higher polyhydric alcohols. Specific examples thereof include glycerol trimyristate, glycerol triisopalmitate, glycerol tri-2-heptylundecanoate, trimethylolpropane triethylhexanoate, trimethylolpropane trioctanoate, glycerol tri-(caprylate·caprate), glycerol trioleate, glycerol tri-2-ethylhexanoate, glycerol triisostearate, olive oil and jojoba oil. Among these, it is preferable to contain at least one or more selected from the group consisting of glycerol tri-(caprylate-caprate), glycerol trioleate, glycerol tri-2-ethylhexanoate and glycerol triisostearate; and it is more preferable to contain glycerol tri-(caprylate-caprate), in view of providing the solid powder cosmetic which is good in the dispersibility of the pigment, has no agglomerate, and is excellent in the glittering property and excellent in use impression.

The tetraester oils include tetra-fatty acid esters of tetra- or higher polyhydric alcohols. Specific examples thereof include pentaerythritol tetra-(behenate/ benzoate/ethylhexanoate), pentaerythritol tetraethylhexanoate, pentaerythritol tetraoctanoate and pentaerythritol tetra-2-ethylhexanoate. Among these, it is preferable to contain at least one or more selected from the group consisting of pentaerythritol tetraethylhexanoate, pentaerythritol tetraoctanoate and pentaerythritol tetra-2-ethylhexanoate; and it is more preferable to contain pentaerythritol tetraethylhexanoate, in view of providing the solid powder cosmetic which is good in the dispersibility of the pigment, has no agglomerate, and is excellent in the glittering property and excellent in use impression.

The ether oils include dialkyl ethers, and specifically include dihexyl ether, dicaprylyl ether and cetyl-1,3-dimethyl butyl ether. Among these, it is preferable to contain at least one or more selected from the group consisting of dicaprylyl ether and cetyl-1,3-dimethyl butyl ether; and it is more preferable to contain cetyl-1,3-dimethyl butyl ether, in view of providing the solid powder cosmetic which is good in the dispersibility of the pigment, has no agglomerate, and is excellent in the glittering property and excellent in use impression.

The silicone oils include crosslinked methylpolysiloxane, network methylpolysiloxane, dimethylpolysiloxane, methyl trimethicone, dimethylcyclopolysiloxane and methylphenylpolysiloxanes such as diphenylsiloxyphenyl trimethicone and higher alcohol-modified organopolysiloxanes. Among these, it is preferable to contain at least one or more selected from the group consisting of dimethylpolysiloxane, methyl trimethicone, dimethylcyclopolysiloxane and methylphenylpolysiloxane; it is more preferable to contain at least one or more selected from the group consisting of dimethylpolysiloxane, methyl trimethicone and methylphenylpolysiloxane; and it is further more preferable to contain at least one or more selected from the group consisting of dimethylpolysiloxane, methyl trimethicone and diphenylsiloxyphenyl trimethicone, in view of providing the solid powder cosmetic which is good in the dispersibility of the pigment, has no agglomerate, and is excellent in the glittering property and excellent in use impression. The silicone oil even more preferably contains a volatile silicone oil.

Examples of the volatile silicone oil include linear dimethylpolysiloxanes such as dimethylpolysiloxane (1 cs), dimethylpolysiloxane (1.5 cs) and dimethylpolysiloxane (2 cs); branched siloxanes such as methyl trimethicone, tris(trimethylsilyl)methylsilane and tetrakis(trimethylsilyl)silane; and cyclic dimethylsiloxanes such as octamethylcyclotetrasiloxane, decamethylcyclopentasiloxane and dodecamethylcyclohexasiloxane; it is preferable to contain at least one or more selected from the group consisting of linear dimethylpolysiloxanes and branched siloxanes; and it is more preferable to contain at least one or more selected from the group consisting of dimethylpolysiloxane (1.5 cs) and methyl trimethicone.

With regard to the higher alcohols having 10 to 24 carbon atoms, it is preferable to contain at least one or more selected from the group consisting of higher alcohols having 12 to 22 carbon atoms; it is more preferable to contain at least one or more selected from the group consisting of higher alcohols having 16 to 22 carbon atoms; it is further more preferable to contain at least one or more selected from the group consisting of higher alcohols having 18 to 22 carbon atoms; and it is even more preferable to contain at least one or more selected from the group consisting of higher alcohols having 18 or 22 carbon atoms, in view of providing the solid powder cosmetic which is good in the dispersibility of the pigment, has no agglomerate, and is excellent in the glittering property and excellent in use impression.

With regard to the oil agent, it is preferable to contain at least one or more selected from the group consisting mineral oils, ester oils and silicone oils; it is more preferable to contain at least one or more selected from the group consisting of liquid paraffin among mineral oils, monoester oils, diester oils and silicone oils; and it is further more preferable to contain at least one or more selected from the group consisting of liquid paraffin, monoester oils, diester oils, dimethylpolysiloxane, methyl trimethicone and methylphenylpolysiloxane, in view of providing the solid powder cosmetic which is good in the dispersibility of the pigment, has no agglomerate, and is excellent in the glittering property and excellent in use impression.

The oil agent is preferably one which is liquid at 25°C in view of providing the solid powder cosmetic which is good in the dispersibility of the pigment, has no agglomerate, and is excellent in the glittering property and excellent in use impression. The oil agent more preferably contains at least the volatile silicone oil.

The content of the oil agent in the solid powder cosmetic can suitably be regulated in consideration of the use impression, the moldability and the impact resistance, and is preferably, for example, 5 mass% or higher and 30 mass% or lower, more preferably 7 mass% or higher, further more preferably 10 mass% or higher, even more preferably 12 mass% or higher, even more preferably 14 mass% or higher and even more preferably 16 mass% or higher, and then, more preferably 28 mass% or lower, further more preferably 25 mass% or lower and even more preferably 22 mass% or lower.

Herein, the content of a component means the mass proportion of the component to the substance(s) as a basis, the mass of which is regarded as 100, and is expressed in "mass%" as unit.

It is preferable that the powder to be used for the present invention contains a glittering pigment; and it is more preferable that the powder contains a color pigment and a glittering pigment. It is further more preferable that the powder contains a color pigment, a glittering pigment and an extender pigment.

As the glittering pigment, there can be used a platy powder or the like of mica, synthetic phlogopite, glass, silica, alumina or the like coated with a coloring agent such as titanium oxide, iron oxide, silicon oxide, Berlin blue, chromium oxide, tin oxide, chromium hydroxide, gold, silver, carmine or an organic pigment, on their surface; a powder of a raw film cut into an optional shape, such as a polyethylene terephthalate-polymethyl methacrylate laminated powder, a polyethylene terephthalate·aluminum-deposited powder or a polyethylene terephthalate-gold-deposited powder; or the like.

Examples of the color pigment include metal oxides such as titanium oxide, zinc oxide, yellow iron oxide, red iron oxide, black iron oxide, Berlin blue, ultramarine blue, chrome oxide and chromium hydroxide; metal complexes such as manganese violet and cobalt titanate; inorganic pigments such as carbon black; synthetic organic pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No.

204, Orange No. 205, Yellow No. 4, Yellow No. 5, Yellow No. 401, Blue No. 1 and Blue No. 404; and natural organic coloring matter such as β-carotene, caramel and paprika coloring matter.

Examples of the extender pigment include inorganic pigments such as silicic acid, silicic acid anhydride, magnesium silicate, talc, sericite, boron nitride, mica, synthetic mica, glass flake, synthetic phlogopite, kaolin, clay, bentonite, bismuth oxychloride, zirconium oxide, magnesium oxide, aluminum oxide, calcium sulfate, barium sulfate, magnesium sulfate, calcium carbonate, magnesium carbonate, aluminum silicate, magnesium silicate, silica and alumina, and composite powders of these.

Further, there can be used an organic powder such as a polyethylene powder, polypropylene powder, polymethyl methacrylate powder, nylon powder, polytetrafluoroethylene powder, silicone powder, silicone rubber powder, silk powder, urethane powder, cellulose powder, starch powder or polyfluoroethylene powder; an inorganic powder such as silica, magnesium carbonate or calcium carbonate; acylated lysine powder such as lauroyl lysine; a metallic soap powder being a higher fatty acid metal salt; or the like.

Then, the above-mentioned organic powders and inorganic powders are preferably globular powders in view of appropriately taking the powder of the solid powder cosmetic and favorably spreading to skin, and more preferred is combination use of the globular powder with at least any one pigment among the extender pigment, the color pigment and the glittering pigment.

These powders are not limited in terms of the size, the shape and the like. They can be used as they are, or can be subjected to hydrophobization treatment or hydrophilization treatment by usual methods, before use.

The hydrophobization treatment is not limited as long as it is a treatment usually applied to powders for cosmetics, and may be carried out as a dry treatment, a wet treatment or the like by using a surface treating agent such as a fluorocompound, silicone-based compound, metallic soap, amino acid-based compound, lecithin, alkyl silane, oil agent, or organic titanate.

Specific examples of the surface treating agent include fluorocompounds such as perfluoropolyether, perfluoroalkyl phosphates, perfluoroalkylalkoxysilane and fluorine-modified silicones; silicone-based compounds such as dimethylpolysiloxane, methylhydrogenpolysiloxane, cyclic silicones, one-end or both-end trialkoxy group-modified organopolysiloxane, crosslinked silicone, silicone resins, fluorine-modified silicone resins and acryl-modified silicone; metallic soaps such as aluminum stearate, aluminum myristate, zinc stearate and magnesium stearate; amino acid-based compounds such as proline, hydroxyproline, alanine, glycine, sarcosine, glutamic acid, aspartic acid, lauroyllysine, lysine and derivatives of these, and acylated amino acids; lecithin and hydrogenated lecithin; alkylsilanes such as methyltrimethoxysilane, ethyltrimethoxysilane, hexyltrimethoxysilane, octyltrimethoxysilane, octyltriethoxysilane and triethoxycaprylylsilane; oil agents such as polyisobutylene, waxes, fats and oils and fatty acids; and organic titanates such as isopropyl triisostearoyl titanate.

Then, the hydrophilization treatment is not limited as long as it is a treatment usually applied to powders for cosmetics.

Examples therefor include plant-derived polymers such as gum arabic, gum tragacanth, arabinogalactan, locust bean gum (carob gum), guar gum, karaya gum, carrageenan, pectin, agar, quince seed (marmelo), starch (rice, corn, potato, wheat), algae colloid, tranto gum and locust bean gum; microbial polymers such as xanthan gum, dextran, succinoglycan and pullulan; animal-derived polymers such as collagen, casein, albumin, deoxyribonucleic acids (DNA), and salts thereof; starch-based polymers such as carboxymethyl starch and methylhydroxypropyl starch; cellulose-based polymers such as methylcellulose, ethylcellulose, methylhydroxypropylcellulose, carboxymethylcellulose, hydroxymethylcellulose, hydroxypropylcellulose, nitrocellulose, sodium cellulose sulfate, sodium carboxymethylcellulose, crystalline cellulose and cellulose powders; alginic acid-based polymers such as sodium alginate and propylene glycol alginate; vinyl-based polymers such as polyvinyl methyl ether, polyvinylpyrrolidone, carboxyvinyl polymers; polyoxyethylene-based polymers such as polyethylene glycol and polyethylene glycol silane; polyoxyethylene polyoxypropylene copolymer-based polymers; acrylic polymers such as sodium polyacrylate, polyethyl acrylate and polyacrylic acid amide; and further, inorganic silicate-based compounds such as silica.

In view of taking the powder, the spreadability while applying, and the makeup persistency, the powder is preferably one containing a hydrophobic powder, and more preferably one surface-treated with a fluoro compound, silicone-based compound, metallic soap, amino acid-based compound, lecithin, alkylsilane, oil agent, organic titanate or the like.

In the case of containing the above-mentioned hydrophobic powder as a powder, the content of the hydrophobic powder is, in the whole powder, preferably 10 mass% or higher, more preferably 30 mass% or higher and further more preferably 50 mass% or higher.

The powder can be used singly or in a combination of two or more thereof; and the total content of the powder in the solid powder cosmetic of the present invention can be regulated in any content in view of the color tone, the use impression and the moldability, and is preferably 70 mass% or higher and 95 mass% or lower, more preferably 73 mass% or higher and 90 mass% or lower, further more preferably 75 mass% or higher and 87 mass% or lower and even more preferably 75 mass% or higher and 84 mass% or lower, in the total amount of the solid powder cosmetic.

The content of the glittering pigment can be regulated in any content in view of the color tone, the use impression and the moldability, and is preferably 10 mass% or higher and 50 mass% or lower, more preferably 15 mass% or higher and 45 mass% or lower and further more preferably 20 mass% or higher and 40 mass% or lower, in the total amount of the solid powder cosmetic.

The content of the color pigment can be regulated in any content in view of the color tone, the use impression and the moldability, and is preferably 2 mass% or higher and 30 mass% or lower, more preferably 5 mass% or higher and 25 mass% or lower and further more preferably 5 mass% or higher and 20 mass% or lower, in the total amount of the solid powder cosmetic.

The content of the extender pigment can be regulated in any content in view of the color tone, the use impression and the moldability, and is preferably 10 mass% or higher and 60 mass% or lower, more preferably 15 mass% or higher and 55 mass% or lower and further more preferably 20 mass% or higher and 50 mass% or lower, in the total amount of the solid powder cosmetic.

The solvent to be used in the step (A) may be any aqueous solvent having an SP value of 19 $(cal/cm^3)^{1/2}$ or higher, but is preferably an aqueous solvent having an SP value of 20 $(cal/cm^3)^{1/2}$ or higher, more preferably an aqueous solvent having an SP value of 21 $(cal/cm^3)^{1/2}$ or higher and further more preferably an aqueous solvent having an SP value of 22 $(cal/cm^3)^{1/2}$ or higher, in view of leaving the oil agent sufficiently in products.

The SP value is a physical property value defined as a square root of the cohesive energy density, and is a numerical value indicating the solubility behavior of the solvent. The SP value (theoretical value) of water is 23.4; the SP value (theoretical value) of n-hexane is 7.3; and the SP value (theoretical value) of ethanol is 12.7.

In the present invention, in view of leaving the oil agent sufficiently in products, it is preferable to use an aqueous solvent having an SP value that does not dissolve the oil agent completely; and it is preferable to use an aqueous solvent having an SP value of 19 $(cal/cm^3)^{1/2}$ or higher; it is more preferable to use water or a water-ethanol solution having an SP value of 19 or higher; it is further more preferable to use a water-ethanol solution having an SP value of 20 $(cal/cm^3)^{1/2}$ or higher; it is even more preferable to use a water-ethanol solution having an SP value of 21 $(cal/cm^3)^{1/2}$ or higher; it is even more preferable to use a water-ethanol solution having an SP value of 22 $(cal/cm^3)^{1/2}$ or higher; and it is even more preferable to use water alone.

The amount of the above aqueous solvent to be used may be any amount which can make the oil agent and the powder into a slurry, and is, for example, preferably 20 mass% or larger and 200 mass% or smaller, more preferably 40 mass% or larger and 160 mass% or smaller and further more preferably 60 mass% or larger and 140 mass% or smaller with respect to the total amount of the oil agent and the powder.

In the step (A), surfactants, polyhydric alcohols, water-soluble polymers, beauty ingredients, perfumes, preservatives, ultraviolet absorbents, antioxidants and the like may be contained in addition to the oil agent, the powder, and the aqueous solvent. In the case of containing a surfactant, it is preferable that the surfactant is contained in the range of 12 mass% or less with respect to the oil agent.

It is preferable to use the surfactant, in consideration of homogeneously dispersing the oil agent and the powder in the slurry and suppressing the formation of agglomerates. The surfactant preferably contains a nonionic surfactant.

Examples of the surfactant include nonionic surfactants other than silicone-based ones, and silicone-based nonionic surfactants.

The nonionic surfactants other than silicone-based ones are not limited as long as they are ones used for usual cosmetics, and examples thereof include sorbitan fatty acid esters such as sorbitan monoisostearate, glycerol fatty acid esters, polyglycerol fatty acid esters, propylene glycol fatty acid esters, polyethylene glycol fatty acid esters, sucrose fatty acid esters, polyoxyethylene fatty acid esters, polyoxyethylene sorbitan fatty acid esters, polyoxyethylene alkyl ethers, polyoxyethylene sorbitol fatty acid esters, polyoxyethylene glycerol fatty acid esters, polyoxyethylene propylene glycol fatty acid esters, polyoxyethylene castor oils, polyoxyethylene hydrogenated castor oils, polyoxyethylene hydrogenated castor oil fatty acid esters, polyoxyethylene phytostanol ethers, polyoxyethylene phytosterol ethers, polyoxyethylene cholestanol ethers and polyoxyethylene cholesteryl ethers.

The silicone-based nonionic surfactants include linear or branched polyether-modified silicones such as polyoxyethylene-methylpolysiloxane copolymers and polyoxyethylene-polydimethylsiloxyethyl dimethicone (specifically, PEG-9 polydimethylsiloxyethyl dimethicone); alkyl-modified polyether-modified silicones (specifically, cetyl PEG/PPG-10/1 dimethicone); and alkyl chain-comodified polyether-modified silicones such as alkyl-modified polyoxyethylene·polydimethylsiloxyethyl dimethicone (specifically, lauryl PEG-9 polydimethylsiloxyethyl dimethicone).

Among these, it is preferable to contain at least one or more selected from the group consisting of sorbitan fatty acid esters, linear or branched polyether-modified silicones, alkyl-modified polyether-modified silicones and alkyl-modified polyoxyethylene-polydimethylsiloxylethyl dimethicones; it is more preferable to contain at least one or more selected from the group consisting of sorbitan fatty acid esters, linear or branched polyether-modified silicones and alkyl-modified polyether-modified silicones; and it is further more preferable to contain at least one or more selected from the group consisting of sorbitan fatty acid esters and alkyl-modified polyether-modified silicones, in view of improving the stability of the slurry. Further, it is preferable to use the nonionic surfactant other than silicone-based ones together with the silicone-based nonionic surfactant in combination.

With regard to these nonionic surfactants, the HLB is preferably from 3.5 to 16, more preferably from 7 to 14 and further more preferably from 8 to 12, in view of improving the stability of the slurry.

Here, the HLB (Hydrophilic-Lipophilic Balance) indicates a molecular weight of the hydrophilic group moiety in the entire molecular weight of a surfactant, and is determined by Griffin equation in the case of a nonionic surfactant. The HLB of a mixed surfactant constituted of two or more of nonionic surfactants is determined as follows. The HLB of the mixed surfactant is a value obtained by arithmetically averaging HLB values of each nonionic surfactant based on their blend ratio.

$$\text{Mixed HLB} = \Sigma(\text{HLBx} \times \text{Wx})/\Sigma\text{Wx}$$

wherein HLBx represents an HLB value of a nonionic surfactant X; and Wx represents a weight (g) of the nonionic surfactant X having the value of HLBx.

In the case of using a nonionic surfactant other than silicone-based ones together with a silicone-based nonionic surfactant in combination as the surfactant, the mass ratio of the silicone-based nonionic surfactant to the nonionic surfactant other than silicone-based ones is preferably (silicone-based nonionic surfactant)/(nonionic surfactant other than silicone-based ones) = from 0.5 to 20, more preferably from 1 to 10 and further more preferably from 2 to 5, in view of improving the stability of the slurry.

Such a surfactant can be used singly or in a combination of two or more thereof, and it is preferable to use the surfactant, in view of causing the oil agent and the powder to be homogeneously dispersed through emulsification in the slurry and suppressing the formation of agglomerates. On the other hand, when a large amount of the surfactant is contained, when the solvent is removed from the cosmetic in a slurry form in the later step (C), also the oil agent is liable to be simultaneously removed, making it difficult for the oil agent to be left at a large amount in the cosmetic. From the above, the surfactant is used in an amount of preferably 0 mass% or higher and 12 mass% or lower, more preferably 0 mass% or higher and lower than 12 mass%, further more preferably 0 mass% or higher and 10 mass% or lower, even more preferably 0 mass% or higher and 7 mass% or lower, even more preferably 0 mass% or higher and 5 mass% or lower, even more preferably in 0 mass% or higher and 3 mass% or lower, even more preferably 0 mass% or higher and 2 mass% or lower and even more preferably 0 mass%, with respect to the total amount of the oil agent.

In the step (A), in view of being good in the dispersibility of the pigment, having no agglomerates and further improving the glittering property, it is preferable that the oil agent, the powder and the aqueous solvent are mixed to make a slurry without previously mixing the oil agent and the powder. The oil agent, the powder, and the aqueous solvent may be mixed at once; after the oil agent and the aqueous solvent are mixed, the powder may be mixed; or after the powder and the aqueous solvent are mixed, the oil agent may be mixed.

An apparatus to be used in this step suffices so long as being a mixing apparatus capable of homogeneously mixing the oil agent, the powder, and the aqueous solvent and a high-speed dispersing machine, for example, a homo disper having a disper blade, can be used.

The circumferential speed of the stirring blade tip of the mixing apparatus in the mixing is, in view of good dispersibility of the pigment and good dispersibility of the oil agent even at a content of the surfactant of 10 mass% or lower with respect to the oil agent, and also in view of preventing crushing of the glittering pigment and reduction of the load in the production, preferably 6 m/s or higher and 39 m/s or lower. Further, in view of the dispersibility (suppression of the formation of agglomerates) of the pigment and the dispersibility of the oil agent, the speed is more preferably 8 m/s or higher, further more preferably 10 m/s or higher, even more preferably 15 m/s or higher and even more preferably 20 m/s or higher; and in view of preventing crushing of the glittering pigment and reduction of the load in the production, the speed is preferably 35 m/s or lower and more preferably 30 m/s or lower.

The stirring time is preferably 1 min or longer, more preferably 3 min or longer and further more preferably 5 min or longer, in view of the dispersibility (suppression of the formation of agglomerates) of the pigment and the dispersibility of the oil agent; and the time is preferably 120 min or shorter, more preferably 75 min or shorter and further more preferably 60 min or shorter in view of preventing crushing of the glittering pigment and reduction of the load in the production.

The stirring temperature is preferably 15°C or higher, more preferably 20°C or higher and further more preferably 25°C or higher, in view of the dispersibility (suppression of the formation of agglomerates) of the pigment, and the dispersibility of the oil agent; and preferably 90°C or lower, more preferably 80°C or lower and further more preferably 70°C or lower in view of the reduction of the load in the production and the safety in the operation.

The step (B) is a step of packing a cosmetic in a slurry form obtained by the step (A) in a cosmetic container.

The cosmetic container to be used for the step (B) is a container suitable for the intended solid powder cosmetic. Examples thereof include resin-made containers of a PET resin, AS resin, ABS resin, polycarbonate resin or the like, and metal-made containers of aluminum, iron or the like.

A packing method is not especially limited, and may be either of front packing and back packing.

The step (C) is a step of, after the step (B), removing the solvent from the cosmetic in the slurry form.

With regard to the timing of the removal of the solvent from the packed cosmetic, the solvent may be removed by suction after the completion of packing of the slurry in the cosmetic container, or the slurry is packed in the cosmetic container while the solvent is removed by suction, but it is preferable to mold the slurry by press in the cosmetic container while the solvent is removed by suction. Means of removing the solvent may be vacuum suction and, besides, may be removal by absorption by using a porous material, cloth or paper.

Here, the degree of vacuum in removal of the solvent by suction is preferably -5 kPa or higher and 80 kPa or lower, in view of the efficiency of the removal of the solvent and the load of the apparatus in the production.

Further, it is preferable that the solid powder cosmetic be dried, as required. Drying means is not especially limited, and may be natural drying, and hot air drying and vacuum drying can preferably be used.

In view of the drying efficiency, the load of the apparatus in the production and the thermal degradation of products, the drying temperature is preferably 20°C or higher, more preferably 25°C or higher and further more preferably 30°C or higher, and preferably 95°C or lower, more preferably 90°C or lower and further more preferably 85°C or lower.

The amount of moisture (moisture content) in the solid powder cosmetic after drying is preferably from 0.01 to 1.0 mass%, more preferably from 0.05 to 0.8 mass% and further more preferably from 0.1 to 0.5 mass%, in view of the amount taken upon application and the drop-resistant strength.

Examples of the solid powder cosmetic obtained by the method of the present invention includes makeup cosmetics such as eye colors, foundations, cheek blushes, eye liners, face powders and lip sticks.

With regard to the above-mentioned embodiments, the present invention further discloses the following production methods.

<1> A method for producing a solid powder cosmetic, the method comprising the following steps (A) to (C):

(A) a step of mixing an oil agent, a powder, an aqueous solvent having an SP value of 22 $(cal/cm^3)^{1/2}$ or higher, and a surfactant in an amount of 0 to 7 mass% with respect to the oil agent, and stirring the mixture at a circumferential speed of a stirring blade tip of 6 m/s or higher and 39 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<2> A method for producing a solid powder cosmetic, the method comprising the following steps (A) to (C):

(A) a step of mixing an oil agent, a powder, an aqueous solvent having an SP value of 22 $(cal/cm^3)^{1/2}$ or higher, and a surfactant in an amount of 0 to 10 mass% with respect to the oil agent, and stirring the mixture at a circumferential speed of a stirring blade tip of 10 m/s or higher and 30 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<3> A method for producing a solid powder cosmetic, the method comprising the following steps (A) to (C):

(A) a step of mixing an oil agent, a powder, an aqueous solvent having an SP value of 21 $(cal/cm^3)^{1/2}$ or higher, and a surfactant in an amount of 0 to 7 mass% with respect to the oil agent, and stirring the mixture at a circumferential speed of a stirring blade tip of 10 m/s or higher and 30 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<4> A method for producing a solid powder cosmetic, the method comprising the following steps (A) to (C):

(A) a step of mixing an oil agent, a powder, an aqueous solvent having an SP value of 22 $(cal/cm^3)^{1/2}$ or higher, and a surfactant in an amount of 0 to 7 mass% with respect to the oil agent, and stirring the mixture at a circumferential speed of a stirring blade tip of 10 m/s or higher and 30 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<5> A method for producing a solid powder cosmetic, the method comprising the following steps (A) to (C):

(A) a step of mixing an oil agent, a powder comprising a color pigment and a glittering pigment, an aqueous solvent having an SP value of 21 $(cal/cm^3)^{1/2}$ or higher, and a surfactant in an amount of 0 to 10 mass% with respect to the oil agent, and stirring the mixture at a circumferential speed of a stirring blade tip of 6 m/s or higher and 39 m/s or lower for 5 to 60 min to thereby make a slurry;
(B) a step of packing a cosmetic in a slurry form obtained in the step (A) in a cosmetic container; and
(C) a step of, after the step (B), removing the solvent from the cosmetic in the slurry form.

<6> A method for producing a solid powder cosmetic, the method comprising the following steps (A) to (C):

(A) a step of mixing an oil agent, a powder, an aqueous solvent having an SP value of 21 $(cal/cm^3)^{1/2}$ or higher, and a surfactant in an amount of 0 to 10 mass% with respect to the oil agent without previously mixing the oil agent and the powder, and stirring the mixture at a circumferential speed of a stirring blade tip of 6 m/s or higher and 39 m/s or lower for 5 to 60 min to thereby make a slurry;
(B) a step of packing a cosmetic in a slurry form obtained in the step (A) in a cosmetic container; and
(C) a step of, after the step (B), removing the solvent from the cosmetic in the slurry form.

<7> A method for producing a solid powder cosmetic, the method comprising the following steps (A) to (C):

(A) a step of mixing an oil agent, a powder comprising a color pigment and a glittering pigment, an aqueous solvent having an SP value of 21 $(cal/cm^3)^{1/2}$ or higher, and a surfactant in an amount of 0 to 10 mass% with respect to the oil agent without previously mixing the oil agent and the powder, and stirring the mixture at a circumferential speed of a stirring blade tip of 6 m/s or higher and 39 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<8> A method for producing a solid powder cosmetic, the method comprising the following steps (A) to (C):

(A) a step of mixing an oil agent, a powder comprising a color pigment and a glittering pigment, an aqueous solvent having an SP value of 21 $(cal/cm^3)^{1/2}$ or higher, and a surfactant in an amount of 0 to 10 mass% with respect to the oil agent without previously mixing the oil agent and the powder, and stirring the mixture at a circumferential speed of a stirring blade tip of 6 m/s or higher and 39 m/s or lower for 5 to 60 min to thereby make a slurry;
(B) a step of packing a cosmetic in a slurry form obtained in the step (A) in a cosmetic container; and
(C) a step of, after the step (B), removing the solvent from the cosmetic in the slurry form.

<9> A method for producing a solid powder cosmetic, the method comprising the following steps (A) to (C):

(A) a step of mixing an oil agent, a powder comprising a color pigment and a glittering pigment, an aqueous solvent having an SP value of 22 $(cal/cm^3)^{1/2}$ or higher, and a surfactant in an amount of 0 to 7 mass% with respect to the oil agent without previously mixing the oil agent and the powder, and stirring the mixture at a circumferential speed of a stirring blade tip of 10 m/s or higher and 30 m/s or lower for 5 to 60 min to thereby make a slurry;
(B) a step of packing a cosmetic in a slurry form obtained in the step (A) in a cosmetic container; and
(C) a step of, after the step (B), removing the solvent from the cosmetic in the slurry form.

<10> A method for producing a solid powder cosmetic, the method comprising the following steps (A) to (C):

(A) a step of mixing an oil agent, a powder, and an aqueous solvent having an SP value of 19 $(cal/cm^3)^{1/2}$ or higher, and stirring the mixture at a circumferential speed of a stirring blade tip of 6 m/s or higher and 39 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

<11> A method for producing a solid powder cosmetic, the method comprising the following steps (A) to (C):

(A) a step of mixing an oil agent, a powder, and an aqueous solvent having an SP value of 20 $(cal/cm^3)^{1/2}$ or higher, and stirring the mixture at a circumferential speed of a stirring blade tip of 10 m/s or higher and 30 m/s or lower to thereby make a slurry;
(B) a step of packing a cosmetic in a slurry form obtained in the step (A) in a cosmetic container; and

(C) a step of, after the step (B), removing the solvent from the cosmetic in the slurry form.

<12> A method for producing a solid powder cosmetic, the method comprising the following steps (A) to (C):

(A) a step of mixing an oil agent, a powder, an aqueous solvent having an SP value of 19 $(cal/cm^3)^{1/2}$ or higher, and a surfactant in an amount of 0 to 12 mass% with respect to the oil agent without previously mixing the oil agent and the powder, and stirring the mixture at a circumferential speed of a stirring blade tip of 10 m/s or higher and 30 m/s or lower to thereby make a slurry;
(B) a step of packing a cosmetic in a slurry form obtained in the step (A) in a cosmetic container; and
(C) a step of, after the step (B), removing the solvent from the cosmetic in the slurry form.

Examples

Then, the present invention will be described in more detail by way of Examples, but the present invention is not any more limited to these Examples.

[Method of preparing molded products]

Examples 1 to 15 and Comparative Examples 1 to 8

A solvent and an oil agent in amounts described in Table 1 were charged in a beaker and stirred by a disper of 40 mm for 1 min; and thereafter, a pigment was added thereto; and the resultant was mixed at a predetermined rotation speed by using the disper at 25°C for 10 min to thereby obtain a slurry.
3 g of the obtained slurry was packed in a metal dish with a size of 27 mm × 27 mm, and twice pressed at a pressing pressure of 1 kN for 2 sec, with two sheets of cloth interposed between a pressing plate having a suction hole and the metal dish, while the solvent was removed by suction through the suction hole by vacuum. Thereafter, the resultant was dried at 40°C for 24 hours to thereby prepare a solid powder cosmetic.

[Method of measuring the oil agent amount in molded products (before drying)]

By using an EXSTAR6000 (manufactured by Hitachi High-Tech Science Corporation), 10 mg of a molded product was heated up to 600°C and heated until the mass thereof did not change, and the oil agent amount was calculated from a change in the mass.

((A mass before heating) − (a mass after heating) + (1) + (2))/(the mass before heating)

(1) A change in mass when drying was carried out at 105°C for 3 hours (assuming moisture contained)
(2) A change in mass when a pigment alone was heated by the same method (assuming volatile components in the pigment)

[Method of sensory evaluation of the molded products (after drying)]

Five cosmetics researchers evaluated the "agglomerates of the color pigment by visual observation", the "intensity of glittering of the glittering pigment by visual observation" and the "smoothness upon application" of the prepared solid powder cosmetics, on the basis of a perfect score of 5 points; and the evaluation was made in point scores (rounded point scores) of the solid powder cosmetics.

[Agglomerates of the color pigment by visual observation]

5 points: there were agglomerates in the state of agglomerates of Example 7.
4 points: as compared with Example 7, there were slightly much agglomerates.

3 points: as compared with Example 7, there were much agglomerates.
2 points: as compared with Comparative Example 1, there were little agglomerates.
1 point: as compared with Comparative Example 1, there were agglomerates in the state of agglomerates of Comparative Example 1.

[Intensity of glittering of the glittering pigment by visual observation]

5 points: the glittering was glittering of the glittering pigment of Comparative Example 1.
4 points: as compared with Comparative Example 1, the glittering of the glittering pigment was quite slightly weak.
3 points: as compared with Comparative Example 1, the glittering of the glittering pigment was slightly weak.
2 points: as compared with Comparative Example 2, the glittering of the glittering pigment was slightly strong.
1 point: the glittering was glittering of the glittering pigment of Comparative Example 2.

[Moist smoothness upon application]

5 points: the feeling upon application was very moist and smooth (Example 4).
4 points: the moist smooth feeling upon application was quite slightly inferior to Example 4.
3 points: the moist smooth feeling upon application was slightly inferior to Example 4.
2 points: the moist smooth feeling upon application was inferior to Example 4.
1 point: the feeling upon application was dry and crumbly and the feeling of foreign matters was perceived (Comparative Example 4).

There are shown in Table 1, formulations, kinds and SP values of the solvents, and production conditions and evaluation results of the molded products.

[Table 1]

| | Example | | | | | | | | | | | | | | | Comparative Example | | | | | | | |
|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 1 | 2 | 3 | 4 | 5 | 6 | 7 | 8 |
| Blend Composition [g] / Powder / Extender Pigment / Cericite*1 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 |
| Blend Composition [g] / Powder / Extender Pigment / Talc*2 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 |
| Blend Composition [g] / Powder / Color Pigment / Black iron oxide*3 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 | 3.00 |
| Blend Composition [g] / Powder / Glittering Pigment / Titanium oxide-coated synthetic mica*4 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 | 9.00 |
| Blend Composition [g] / Oil Agent / Mineral oil*5 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 | 6.00 |
| Blend Composition [g] / Surfactant / Polyoxyethylene sorbitol tetraoleate*6 | 0.00 | 0.00 | 0.00 | 0.00 | 0.00 | 0.00 | 0.00 | 0.00 | 0.00 | 0.03 | 0.06 | 0.12 | 0.30 | 0.48 | 0.60 | 0.00 | 0.00 | 0.00 | 0.00 | 0.00 | 0.00 | 0.67 | 0.67 |
| Blend Composition [g] / Surfactant / vs. oil agent [mass%] | 0.00 | 0.00 | 0.00 | 0.00 | 0.00 | 0.00 | 0.00 | 0.00 | 0.00 | 0.50 | 1.00 | 2.00 | 5.00 | 8.00 | 10.00 | 0.00 | 0.00 | 0.00 | 0.00 | 0.00 | 0.00 | 11.10 | 11.10 |
| Blend Composition [g] / Solvent / Amount | 30.00 | 30.00 | 30.00 | 30.00 | 30.00 | 30.00 | 30.00 | 30.00 | 30.00 | 29.97 | 29.94 | 29.88 | 29.70 | 29.52 | 29.40 | 30.00 | 30.00 | 30.00 | 30.00 | 30.00 | 30.00 | 29.33 | 29.33 |
| Blend Composition [g] / Total | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 | 60.00 |
| Solvent Properties / Kind | Distilled water | Distilled water | Distilled water | Distilled water | Distilled water | Distilled water | Distilled water | 10% EtOH | 20% EtOH | Distilled water | Distilled water | Distilled water | Distilled water | Distilled water | Distilled water | Distilled water | Distilled water | 30% EtOH | 40% EtOH | 50% EtOH | 95% EtOH | Distilled water | Distilled water |
| Solvent Properties / SP value [(cal/cm³)^1/2] | 23.4 | 23.4 | 23.4 | 23.4 | 23.4 | 23.4 | 23.4 | 22.4 | 21.3 | 23.4 | 23.4 | 23.4 | 23.4 | 23.4 | 23.4 | 23.4 | 23.4 | 20.3 | 19.3 | 18.2 | 12.9 | 23.4 | 23.4 |
| Production Condition / Circumferential speed of stirring blade tip [m/s] | 8 | 10 | 15 | 20 | 25 | 30 | 35 | 30 | 30 | 20 | 20 | 20 | 20 | 20 | 20 | 5 | 40 | 30 | 30 | 30 | 30 | 5 | 20 |
| Component Composition in Cosmetic after Production [mass%] / Powder | 80.96 | 80.97 | 80.82 | 80.83 | 80.80 | 80.32 | 80.33 | 82.50 | 84.59 | 82.82 | 83.06 | 83.01 | 84.02 | 85.29 | 85.47 | 81.31 | 80.47 | 86.23 | 88.58 | 91.64 | 95.21 | 86.74 | 87.00 |
| Component Composition in Cosmetic after Production [mass%] / Oil agent | 18.82 | 18.82 | 18.99 | 18.99 | 18.99 | 19.48 | 19.48 | 17.32 | 15.23 | 17.07 | 16.82 | 16.89 | 15.87 | 14.60 | 14.42 | 18.50 | 19.31 | 13.58 | 11.29 | 8.25 | 4.76 | 13.15 | 12.89 |
| Component Composition in Cosmetic after Production [mass%] / Moisture content rate | 0.22 | 0.21 | 0.19 | 0.18 | 0.21 | 0.20 | 0.19 | 0.18 | 0.18 | 0.11 | 0.12 | 0.10 | 0.11 | 0.11 | 0.11 | 0.19 | 0.22 | 0.19 | 0.13 | 0.11 | 0.03 | 0.11 | 0.11 |
| Component Composition in Cosmetic after Production [mass%] / Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Evaluations / Remaining amount of oil agent [mass%] | 93 | 93 | 94 | 94 | 94 | 97 | 97 | 84 | 72 | 83 | 81 | 82 | 76 | 70 | 69 | 91 | 96 | 63 | 51 | 36 | 20 | 62 | 61 |
| Evaluations / Agglomerate of color pigment | 2 | 3 | 4 | 5 | 5 | 5 | 5 | 5 | 5 | 5 | 5 | 5 | 5 | 5 | 5 | 1 | 5 | 5 | 5 | 5 | 5 | 3 | 5 |
| Evaluations / Intensity of glittering of glittering pigment | 5 | 5 | 5 | 4 | 4 | 3 | 2 | 3 | 3 | 4 | 4 | 4 | 3 | 3 | 3 | 5 | 1 | 3 | 3 | 3 | 3 | 4 | 2 |
| Evaluations / Moist smoothness upon application | 3 | 4 | 4 | 5 | 5 | 4 | 4 | 4 | 4 | 5 | 5 | 5 | 4 | 4 | 3 | 2 | 3 | 3 | 3 | 1 | 1 | 3 | 3 |

*1: Sericite FSE (Sanshin Koko KK)

*2: Talc JA-68R (Asada Milling Co. Ltd.)

*3: synthetic iron oxide BL-100P (Titan Kogyo, Ltd.)

*4: Flamenco Super Pearl (BASF SE)

*5: mineral oil (Sonneborn LLC)

*6: Rheodol 430V LOT2085 (Kao Corp.)

From Table 1, it is clear that by adopting the step (A) of mixing an oil agent, a powder, an aqueous solvent having an SP value of 21 $(cal/cm^3)^{1/2}$ or higher, and a surfactant in an amount of 0 to 10 mass% with respect to the oil agent, and mixing the mixture at a circumferential speed of a stirring blade tip of 6 m/s or higher and 39 m/s or lower to thereby make a slurry, there could be produced the solid powder cosmetic which simultaneously satisfied both the dispersibility and the glittering property of the pigment, and was excellent in use impression.

## Claims

1. A method for producing a solid powder cosmetic, the method comprising the following steps (A) to (C):

   (A) a step of mixing an oil agent, a powder, an aqueous solvent having an SP value of 21 $(cal/cm^3)^{1/2}$ or higher, and a surfactant in an amount of 0 to 10 mass% with respect to the oil agent, and mixing the mixture at a circumferential speed of a stirring blade tip of 6 m/s or higher and 39 m/s or lower to thereby make a slurry;
   (B) a step of packing a cosmetic in a slurry form obtained in the step (A) in a cosmetic container; and
   (C) a step of, after the step (B), removing the solvent from the cosmetic in the slurry form.

2. The method for producing a solid powder cosmetic according to claim 1, wherein the powder comprises a glittering pigment.

3. The method for producing a solid powder cosmetic according to claim 1 or 2, wherein the powder comprises a color pigment and a glittering pigment.

4. The method for producing a solid powder cosmetic according to any one of claims 1 to 3, wherein the circumferential speed of the stirring blade tip is 8 m/s or higher and 35 m/s or lower.

5. The method for producing a solid powder cosmetic according to any one of claims 1 to 4, wherein the circumferential speed of the stirring blade tip is 10 m/s or higher and 30 m/s or lower.

6. The method for producing a solid powder cosmetic according to any one of claims 1 to 5, wherein the amount of the surfactant to be used is 0 to 7 mass% with respect to the oil agent.

7. The method for producing a solid powder cosmetic according to any one of claims 1 to 6, wherein the SP value of the aqueous solvent is 22 $(cal/cm^3)^{1/2}$ or higher.

8. The method for producing a solid powder cosmetic according to any one of claims 1 to 7, wherein a temperature for the mixing is 15°C or higher and 90°C or lower.

9. The method for producing a solid powder cosmetic according to any one of claims 1 to 8, wherein a time for the mixing is 1 min or longer and 120 min or shorter.

10. The method for producing a solid powder cosmetic according to any one of claims 1 to 9, wherein the step (A) is a step of mixing the oil agent, the powder, and the aqueous solvent without previously mixing the oil agent and the powder to thereby make a slurry.

## INTERNATIONAL SEARCH REPORT

International application No.
**PCT/JP2022/001135**

**A. CLASSIFICATION OF SUBJECT MATTER**

***A61K 8/02***(2006.01)i; ***A61K 8/19***(2006.01)i; ***A61K 8/25***(2006.01)i; ***A61K 8/31***(2006.01)i; ***A61Q 1/12***(2006.01)i
FI: A61K8/02; A61K8/19; A61K8/25; A61K8/31; A61Q1/12

According to International Patent Classification (IPC) or to both national classification and IPC

**B. FIELDS SEARCHED**

Minimum documentation searched (classification system followed by classification symbols)

A61K8/02; A61K8/19; A61K8/25; A61K8/31; A61Q1/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-183352 A (KAO CORP) 12 November 2020 (2020-11-12) claims, paragraphs [0010], [0041], [0045]-[0052] | 1-10 |
| Y | JP 2016-185912 A (NIPPON COKE & ENG CO LTD) 27 October 2016 (2016-10-27) claims, paragraphs [0023], [0036]-[0037], [0046] | 1-10 |
| A | JP 2017-178903 A (NARIS COSMETICS CO LTD) 05 October 2017 (2017-10-05) claims, paragraph [0024] | 1-10 |
| A | JP 2018-016576 A (TOKIWA CORP) 01 February 2018 (2018-02-01) claims, paragraph [0072] | 1-10 |
| A | JP 2014-141483 A (KAO CORP) 07 August 2014 (2014-08-07) claims, paragraphs [0043]-[0059] | 1-10 |

☐ Further documents are listed in the continuation of Box C.   ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 March 2022** | **22 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/001135**

| Patent document cited in search report | Publication date (day/month/year) | Patent family member(s) | Publication date (day/month/year) |
|---|---|---|---|
| JP 2020-183352 A | 12 November 2020 | (Family: none) | |
| JP 2016-185912 A | 27 October 2016 | (Family: none) | |
| JP 2017-178903 A | 05 October 2017 | (Family: none) | |
| JP 2018-016576 A | 01 February 2018 | US 2018/0028414 A1<br>claims, paragraph [0098]<br>FR 3054436 A1<br>CN 107661280 A | |
| JP 2014-141483 A | 07 August 2014 | WO 2014/104348 A1<br>TW 201429495 A<br>CN 104902868 A<br>KR 10-2015-0100639 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001213722 A
- JP 2013209316 A